# EUROPEAN PATENT SPECIFICATION

(11) **EP 0 524 220 B1**
(45) Date of publication and mention of the grant of the patent: **09.11.1994**
(21) Application number: 91907253.8
(22) Date of filing: 21.03.1991
(51) Int. Cl.: D21C 9/00, D21C 5/02, C12S 3/08

(54) **A PULPING PROCESS USING CELLULASE**
EIN VERFAHREN ZUR STOFFAUFBEREITUNG UNTER VERWENDUNG VON CELLULASE
UN PROCEDE DE MISE EN PATE EN UTILISANT DE LA CELLULASE

(30) Priority: 29.03.1990 DK 803/90
(43) Date of publication of application: 27.01.1993
(73) Proprietor: NOVO NORDISK A/S, 2880 Bagsvaerd (DK)
(72) Inventor: BARET, Jean-Luc, Alain, Guy, F-77250 Moret-sur-Loing (FR); LECLERC, Marc, F-92260 Fontenay-aux-Roses (FR)
(74) Representative: Dehmel, Albrecht, Dr. Dipl.-Chem.
(86) International application number: DK9100089
(87) International publication number: WO9114822

(56) References cited:
- EP-A- 0 262 040
- EP-A- 0 334 739
- EP-A- 0 351 655
- GB-A- 2 231 595
- Orbit Search Service, File WPAT, Accession number 88-109831/16, HONSHU PAPER MFG KK, J63059494-A, 880315, 8816, Derwent's abstract
- Orbit Search Service, File WPAT, Accession number 84-051743/09, KAO CORP, J59009299-A, 840118, 8409, Derwent's abstract

## Description

### TECHNICAL FIELD

This invention relates to a pulping process wherein cellulase is used to improve the drainage properties of the pulp.

With reference to GB-A-2 231 595 and DE-A-39 34 772, the applicant has volontarily limited the scope of the present application, and submitted separate claims for the contracting states DE and GB.

### BACKGROUND ART

In the preparation of pulp for paper making, the drainage properties of the pulp may in some cases be unsatisfactory, whereby the capacity of the paper line may be reduced. The drainage properties of the pulp are commonly estimated by the Schopper-Riegler test (a high SR value indicating poor drainage) or by Canadian Standard Freeness (a low CSF value indicating poor drainage). Unsatisfactory drainage may particularly occur in case of repulping material that has already been through a pulping and drying process, e.g. dried virgin pulp, recycled fibre or waste paper.

EP-A-0 262,040 (Cellulose du Pin) and EP-A-0 351,655 (Cultor) describe improved drainability by use of cellulase and hemicellulase during pulping. The consistency (i.e. % dry matter concentration) was 2 - 5%.

J-C Pommier et al., TAPPI Journal, June 1989, pp. 187-191 indicate that 3% consistency is optimum for drainage improvement by cellulase treatment (as indicated by CSF), and that the effect decreases significantly from 3 to 5%.

JP-A-59-9299 and JP-A-63-59494 describe use of cellulase during pulping at 3-6% consistency at high pH (above 9) to improve ink removal from waste paper.

High-consistency pulping at consistency above 8%, typically 10-20%, is commonly used, but use of cellulase in this type of process has not been described.

It is the object of the invention to provide an improved process.

### STATEMENT OF THE INVENTION

We have surprisingly found that drainability can be further improved when the cellulase is used during pulping at high consistency (above 8%).

Accordingly, the invention provides a pulping process wherein cellulase is used to improve the drainage properties of the pulp, characterized by a consistency above 8%.

### DETAILED DESCRIPTION

### Raw material

The process of the invention can be applied to any pulp where it is desired to improve drainability. This is particularly of interest for pulps with SR value above 25 or CSF value below 50, and particularly for repulping of previously pulped and dried material such as dried virgin pulp, recycled fibres and waste paper.

### Cellulase

Many types of cellulase are known and can be used in the process of the invention. Microbial cellulase are preferred for reasons of economy. The cellulase should be active and stable at the conditions, especially the pH, of the process.

Some examples of suitable cellulases are those derived from *Aspergillus* (particularly *A. niger*), *Trichoderma* (particularly *T. viride*, *T. reesei* and *T. koningii*), *Humicola* (particularly *H. insolens*, see US 4,435,307) and alkalophilic *Bacillus* (US 3,844,890). Examples of commercial cellulase preparations are Novozym® 342 (*H. insolens*) and Celluclast 1.5L (*T. reesei*), both available from Novo Nordisk A/S.

A suitable cellulase dosage will usually correspond to a cellulase activity at pH 6 of 100 - 10,000 EGU/kg of dry pulp. Where the pulping process is at alkaline pH (above 7), the cellulase dosage should correspond to a cellulase activity at pH 9 of 100 - 10,000 CEVU/kg of dry pulp.

### Pulping process

The process is carried out at a consistency above 8%, typically 10-20%. The cellulase can be added in an existing high-consistency pulping process using any type of known high-consistency pulper.

Depending e.g. on the use of recycled water, the process may be acidic (e.g. pH 4 - 6) where *Aspergillus* or *Trichoderma* cellulase is preferred. Or, the process pH may be near-neutral (e.g. pH 6 - 8) where *Humicola* or *Bacillus* cellulase is preferred.

When the process of the invention is to be applied to pulping of waste paper, deinking can be achieved by carrying out the pulping at high pH (above 9) in the presence of deinking chemicals (such as sodium hydroxide, sodium silicate, hydrogen peroxide and surfactant), followed by ink separation (e.g. by flotation and/or rinsing). This embodiment of the invention is particularly advantageous since the cellulase will also serve to improve the deinking, while at the same time improving drainage. At the high pH used for deinking, it is preferred to use cellulase from *Humicola* or alkaline *Bacillus*.

The duration of the pulping will generally be 5 - 30 minutes, and this may optionally be followed by maceration (i.e. incubation with or without stirring) to let the enzyme action continue. The temperature throughout this treatment will generally be 15-60°C, typically 30-50°C. The total duration of the cellulase action (i.e. pulping + maceration, if any) will generally be 30-180 minutes.

Pulp prepared according to the invention can be used for conventional paper making.

### EXAMPLES

### Determination of cellulase activity at pH 6 (EGU)

A substrate solution is prepared, containing 34.0 g/l CMC (Hercules 7 LFD) in 0.1 M phosphate buffer at pH 6.0. The enzyme sample to be analyzed is dissolved in the same buffer. 10 ml substrate solution and 0.5 ml enzyme solution are mixed and transferred to a vibration viscosimeter (e.g. MIVI 3000 from Sofraser, France), thermostated at 40°C. One Endo-Glucanase Unit (EGU) is defined as the amount of enzyme that reduces the viscosity to one half under these conditions.

### Determination of cellulase activity at pH 9 (CEVU)

A substrate solution is prepared, containing 33.3 g/l CMC (Hercules 7 LFD) in 0.1 M Tris buffer at pH 9.0. The enzyme sample to be analyzed is dissolved in the same buffer. 10 ml substrate solution and 0.5 ml enzyme solution are mixed and transferred to a viscosimeter (e.g. Haake VT 181, NV sensor, 181 rpm) thermostated at 40°C. One Cellulase Viscosity Unit (CEVU) is defined as the amount of enzyme that reduces the viscosity to one half under these conditions.

### Determination of pulp drainage (Schopper-Riegler)

The Schopper-Riegler number (SR) is determined according to ISO standard 5267 (part 1), on a homogenous pulp at a consistency of 2 g/l. A known volume of pulp is allowed to drain through a metal sieve into a funnel. This funnel has an axial hole and a side hole. The volume of filtrate that has passed through the side hole is measured in a special vessel graduated in Schopper-Riegler units.

### EXAMPLE 1

In this example two types of pulps are pulped at high consistency together with an enzyme treatment by the cellulase preparation Celluclast® 1.5L. The first type of pulp is a raw mechanical pulp from decidous tree, and the second type is a bleached kraft pulp.

The dry pulps are disintegrated in a 20 litres high consistency lab pulper with warm water. The mechanical pulp and the bleached kraft pulp are disintegrated at 8 and 10% consistency, respectively. Their pH is then adjusted to 5.0 with diluted sulfuric acid. The enzyme preparation Celluclast® 1.5L (activity 840 EGU/g) is added to the pulps at the dose of 5 kg/t dry pulp. For each pulp a control experiment without enzyme is also made. In all experiments the temperature is maintained at 45°C +/- 1°C and the pH at 5.0 +/- 0.1. The SR is measured after 90 minutes pulping. The following tables show the resulting SR numbers for both pulps:

| Mechanical pulp | |
|---|---|
| Celluclast® | 60 |
| Control | 67 |
| Difference | 7 |

| Bleached kraft pulp | |
|---|---|
| Celluclast® | 26 |
| Control | 34 |
| Difference | 8 |

The action of the enzyme resulted in a SR decrease of 7 points for the mechanical pulp and 8 points for the bleached kraft pulp.

### EXAMPLE 2

In this example a mix of old cardboard containers is pulped at high consistency together with an enzyme treatment by the cellulase preparation Novozym® 342, at several pH values.

The cardboards are disintegrated at 8% consistency in a 20 litres lab pulper. Their pH is then adjusted to values ranging from 7.6 to 10.1 with diluted sodium hydroxide. The enzyme preparation Novozym® 342(1030 CEVU/g) is added to the pulps at the dose of 5 kg/t dry pulp. For each pulp a control experiment without enzyme is also made. In all experiments the temperature is maintained at 45°C +/- 1°C. The Schopper-Riegler number (SR) is measured after 90 minutes pulping. The following tables show the resulting SR numbers for the different pH values:

| | | |
|---|---|---|
| pH 7.6 | Novozym® 342 | 29 |
| | Control | 38 |
| | Difference | 9 |
| pH 9.1 | Novozym® 342 | 32 |
| | Control | 39 |
| | Difference | 7 |
| pH 9.6 | Novozym® 342 | 33 |
| | Control | 38 |
| | Difference | 5 |
| pH 10.1 | Novozym® 342 | 37 |
| | Control | 38 |
| | Difference | 1 |

The action of Novozym® 342 at various alkaline pH's results in a drainability improvement of 1 to 9 SR points. That can be compared to experiments on the same cardboards where the pulp is incubated with or without the enzyme at 3% consistency instead of 8%. In this case the SR gain provided by the enzyme is only 6 points at pH 7.6 and 2 points at pH 9.1. It is nil at higher pH values. This illustrates the positive effect of high consistency on the SR-reducing action of Novozym® 342 at high pH.

### EXAMPLE 3

In this example three types of pulp are pulped for 20 minutes at high consistency in a 20 litres lab pulper, then macerated without stirring. The enzyme Novozym® 342 is added at the beginning of the pulping. The pulps are raw mechancial pulp, bleached mechanical pulp, and bleached kraft pulp.

Two sets of experiments are made: one at the natural pH of the pulp (7.5), and one at pH 9.5. The pH of the pulp is adjusted with diluted sodium hydroxide at the beginning of the pulping, prior to eventual enzyme addition. The enzyme preparation Novozym® 342 (1030 CEVU/g) is added to the pulps at the dose of 2.5 kg/t dry pulp. For each pulp a control experiment without enzyme is also made. In all experiments the temperature is maintained at 45°C +/- 1°C. The Schopper-Riegler number (SR) is measured after 90 minutes (20 minutes pulping + 70 minutes maceration). The SR results are shown in the following tables:

| Raw mechanical pulp | pH 7.5 | pH 9.5 |
|---|---|---|
| Novozym® 342 | 60 | 63 |
| Control | 70 | 67 |
| Difference | 10 | 4 |

| Bleached mechanical pulp | | |
|---|---|---|
| Novozym® 342 | 57 | 63 |
| Control | 67 | 68 |
| Difference | 10 | 5 |

| Bleached kraft pulp | | |
|---|---|---|
| Novozym® 342 | 23 | 26 |
| Control | 27 | 29 |
| Difference | 4 | 3 |

The enzyme maceration results in a drainability improvement that varies with the pH and the type of pulp.

## Claims (Claims for the following Contracting State(s): AT, BE, CH, DK, ES, FR, GR, IT, LU, NL, SE)

1. A pulping process wherein cellulase is used to improve the drainage properties of the pulp, characterized by a pulp consistency above 8%.

2. A process according to Claim 1 wherein the raw material has an SR value above 25, and is preferably recycled fibre, waste paper or dried virgin pulp.

3. A process according to a preceding claim wherein the cellulase is derived from a strain of *Aspergillus*, *Trichoderm*a, *Humicola* or *Bacillus*.

4. A process according to a preceding claim, wherein the pH is 4-6, and the cellulase is preferably derived from a strain of *Aspergillus* or *Trichoderma*.

5. A process according to any of Claims 1 - 3, wherein the pH is 6-8.5, and the cellulase is preferably derived from a strain of *Humicola* or *Bacillus*.

6. A process for pulping and deinking of waste paper according to any of Claims 1 - 3, wherein the pulping is made at pH 6-9.5 in the presence of deinking chemicals, followed by separation of ink, and wherein the cellulase is preferably derived from *Humicola* or *Bacillus*.

7. A process according to a preceding claim, wherein the amount of cellulase corresponds to 250-5000 CEVU/kg of pulp dry matter.

## Claims (Claims for the following Contracting State(s): DE, GB)

1. A pulping process wherein cellulase is used to improve the drainage properties of the pulp, characterized in that the pulp consistency is above 8% and that the cellulase is derived from a strain of *Aspergillus*, *Trichoderma*, *Humicola* or *Bacillus*.

2. A process according to Claim 1 wherein the raw material has an SR value above 25, and is preferably recycled fibre, waste paper or dried virgin pulp.

3. A process according to a preceding claim, wherein the pH is 4-6, and the cellulase is preferably derived from a strain of *Aspergillus* or *Trichoderma*.

4. A process according to any of Claims 1 - 2, wherein the pH is 6-8.5, and the cellulase is preferably derived from a strain of *Humicola* or *Bacillus*.

5. A process for pulping and deinking of waste paper according to any of Claims 1 - 2, wherein the pulping is made at pH 6-9.5 in the presence of deinking chemicals, followed by separation of ink, and wherein the cellulase is preferably derived from *Humicola* or *Bacillus*.

6. A process according to a preceding claim, wherein the amount of cellulase corresponds to 250-5000 CEVU/kg of pulp dry matter.

## Patentansprüche (Patentansprüche für folgende(n) Vertragsstaat(en): AT, BE, CH, DK, ES, FR, GR, IT, LU, NL, SE)

1. Ein Verfahren zur Stoffaufbereitung, in dem Cellulase verwendet wird, um die Entwässerungseigenschaften des Stoffbreies zu verbessern, gekennzeichnet durch eine Stoffbreikonsistenz über 8%.

2. Ein Verfahren nach Anspruch 1, wobei das Rohmaterial einen SR-Wert über 25 besitzt und vorzugsweise recycelte Faser, Abfallpapier oder getrockneter Rohstoffbrei ist.

3. Ein Verfahren nach einem vorangehenden Anspruch, wobei die Cellulase aus einem Stamm von Aspergillus, Trichoderma, Humicola oder Bacillus gewonnen ist.

4. Ein Verfahren nach einem vorangehenden Anspruch, wobei der pH 4-6 betragt und die Cellulase vorzugsweise aus einem Stamm von Aspergillus oder Trichoderma gewonnen ist.

5. Ein Verfahren nach einem der Ansprüche 1-3, wobei der pH 6-8,5 beträgt und die Cellulase vorzugsweise aus einem Stamm von Humicola oder Bacillus gewonnen ist.

6. Ein Verfahren zur Stoffaufbereitung und zum Deinking von Abfallpapier gemäß einem der Ansprüche 1-3, wobei die Stoffaufbereitung bei pH 6-9,5 in der Gegenwart von Deinking-Chemikalien durchgeführt wird, gefolgt von der Abtrennung des Farbstoffes, und wobei die Cellulase vorzugsweise aus Humicola oder Bacillus gewonnen ist.

7. Ein Verfahren nach einem vorangehenden Anspruch, wobei die Cellulasemenge 250-5.000 CEVU/kg Stoffbrei-Trockensubstanz entspricht.

## Patentansprüche (Patentansprüche für folgende(n) Vertragsstaat(en): DE, GB)

1. Ein Verfahren zur Stoffaufbereitung, in dem Cellulase verwendet wird, um die Entwässerungseigenschaften des Stoffbreies zu verbessern, dadurch gekennzeichnet, daß die Stoffbreikonsistenz über 8% liegt und daß die Cellulase aus einem Stamm von Aspergillus, Trichoderma, Humicola oder Bacillus gewonnen ist.

2. Ein Verfahren nach Anspruch 1, wobei das Rohmaterial einen SR-Wert über 25 besitzt und vorzugsweise recycelte Faser, Abfallpapier oder getrockneter Rohstoffbrei ist.

3. Ein Verfahren nach einem vorangehenden Anspruch, wobei der pH 4-6 beträgt und die Cellulase vorzugsweise aus einem Stamm von Aspergillus oder Trichoderma gewonnen ist.

4. Ein Verfahren nach einem der Ansprüche 1-2, wobei der pH 6-8,5 beträgt und die Cellulase vorzugsweise aus einem Stamm von Humicola oder Bacillus gewonnen ist.

5. Ein Verfahren zur Stoffaufbereitung und zum Deinking von Abfallpapier nach einem der Ansprüche 1-2, wobei die Stoffaufbereitung bei pH 6-9,5 in der Gegenwart von Deinking-Chemikalien durchgeführt wird, gefolgt von der Abtrennung des Farbstoffes, und wobei die Cellulase vorzugsweise aus Humicola oder Bacilius gewonnen ist.

6. Ein Verfahren nach einem vorangehenden Anspruch, wobei die Cellulasemenge 250-5.000 CEVU/kg Stoffbrei-Trockensubstanz entspricht.

## Revendications (Revendications pour l'(les) Etat(s) contractant(s) suivant(s): AT, BE, CH, DK, ES, FR, GR, IT, LU, NL, SE)

1. Procédé de réduction en pâte dans lequel on utilise une cellulase pour améliorer les propriétés d'égouttage de la pâte, caractérisé en ce que la pâte a une consistance supérieure à 8 %.

2. Procédé selon la revendication 1, dans lequel la matière première a une valeur de SR supérieure à 25, et est de préférence constituée par une fibre recyclée, de vieux papiers ou une pâte vierge séchée.

3. Procédé selon l'une des revendications précédentes, dans lequel la cellulase dérive d'une souche d'*Aspergillus*, de *Trichoderm*a, d'*Humicola* ou de *Bacillus*.

4. Procédé selon l'une des revendications précédentes, dans lequel le pH est compris entre 4 et 6, et la cellulase dérive de préférence d'une souche d'*Aspergillus* ou de *Trichoderma*.

5. Procédé selon l'une quelconque des revendications 1 à 3, dans lequel le pH est compris entre 6 et 8,5, et la cellulase dérive de préférence d'une souche d'*Humicola* ou de *Bacillus*.

6. Procédé de réduction en pâte et de désencrage de vieux papiers selon l'une quelconque des revendications 1 à 3, dans lequel on effectue la réduction en pâte à un pH compris entre 6 et 9,5 en présence de produits chimiques de désencrage, puis on sépare l'encre, et dans lequel la cellulase dérive de préférence d'*Humicola* ou de *Bacillus*.

7. Procédé selon l'une des revendications précédentes, dans lequel la quantité de cellulase correspond à 250-5000 CEVU/kg de matière sèche de pâte.

## Revendications (Revendications pour l'(les) Etat(s) contractant(s) suivant(s): DE, GB)

1. Procédé de réduction en pâte dans lequel on utilise une cellulase pour améliorer les propriétés d'égouttage de la pâte, caractérisé en ce que la consistance de la pâte est supérieure à 8 % et en ce que la cellulase dérive d'une souche d'*Aspergillus*, de *Trichoderma*, d'*Humicola* ou de *Bacillus*.

2. Procédé selon la revendication 1, dans lequel la matière première a une valeur de SR supérieure à 25, et est de préférence constituée par une fibre recyclée, de vieux papiers ou une pâte vierge séchée.

3. Procédé selon l'une des revendications précédentes, dans lequel le pH est compris entre 4 et 6, et la cellulase dérive de préférence d'une souche d'*Aspergillus* ou de *Trichoderma*.

4. Procédé selon l'une quelconque des revendications 1 et 2, dans lequel le pH est compris entre 6 et 8,5, et la cellulase dérive de préférence d'une souche d'*Humicola* ou de *Bacillus*.

5. Procédé de réduction en pâte et de désencrage de vieux papiers selon l'une quelconque des revendications 1 et 2, dans lequel on effectue la réduction en pâte à un pH compris entre 6 et 9,5 en présence de produits chimiques de désencrage, puis on sépare l'encre, et dans lequel la cellulase dérive de préférence d'*Humicola* ou de *Bacillus*.

6. Procédé selon l'une des revendications précédentes, dans lequel la quantité de cellulase correspond à 250-5000 CEVU/kg de matière sèche de pâte.
